(19) Europäisches Patentamt
European Patent Office
Office européen des brevets

(11) Veröffentlichungsnummer: **0 008 435**
**B1**

(12) # EUROPÄISCHE PATENTSCHRIFT

(45) Veröffentlichungstag der Patentschrift: **12.05.82**

(51) Int. Cl.³: **C 07 C 157/12,** A 01 N 47/34

(21) Anmeldenummer: **79102966.3**

(22) Anmeldetag: **16.08.79**

(54) **Substituierte N-Phenyl-N'-benzoyl-thioharnstoffe, Verfahren zu ihrer Herstellung und ihre Verwendung als Schädlingsbekämpfungsmittel.**

(30) Priorität: **24.08.78 DE 2837086**

(43) Veröffentlichungstag der Anmeldung: **05.03.80 Patentblatt 80/5**

(45) Bekanntmachung des Hinweises auf die Patenterteilung: **12.05.82 Patentblatt 82/19**

(84) Benannte Vertragsstaaten: **AT BE CH DE FR GB IT NL SE**

(56) Entgegenhaltungen:
**JOURNAL OF AGRICULTURAL AND FOOD CHEMISTRY, Band 24, Nr. 5, September/Oktober 1976, Seiten 1065—1068**
**J. E. OLIVER et al.: »insect Growth Regulators. Analoques of TH-6038 and TH-6040**
***Seite 1066, Formeln 5, 6, 7***

(73) Patentinhaber: **BAYER AG, Zentralbereich Patente, Marken und Lizenzen, D-5090 Leverkusen 1, Bayerwerk (DE)**

(72) Erfinder: **Sirrenberg, Wilhelm, Dr., Wuppertaler Strasse 21, D-4322 Sprockhövel (DE)**
Erfinder: **Klauke, Erich, Dr., Eichendorffweg 8, D-5063 Odenthal (DE)**
Erfinder: **Hammann, Ingeborg, Dr., Belfortstrasse 9, D-5000 Köln 1 (DE)**
Erfinder: **Krehan, Ingomar, Dr., Ludwig-Jahn-Strasse 54, D-5000 Köln 40 (DE)**
Erfinder: **Kraus, Peter, Dr., Düsseldorferstrasse 43, D-5000 Köln 80 (DE)**
Erfinder: **Stendel, Wilhelm, Dr., In den Birken 55, D-56 Wuppertal-1 (DE)**

**EP 0 008 435 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

# Substituierte N-Phenyl-N'-benzoyl-thioharnstoffe, Verfahren zu ihrer Herstellung und ihre Verwendung als Schädlingsbekämpfungsmittel

Die Erfindung betrifft neue substituierte N-Phenyl-N'-benzoyl-thioharnstoffe, ein Verfahren zu ihrer Herstellung sowie ihre Verwendung als Schädlingsbekämpfungsmittel.

Es ist bereits bekannt, daß bestimmte substituierte Benzoyl-thioharnstoffe, wie z. B. N-(2,6-Dichlorbenzoyl)-N'-(3,4-dichlorphenyl)-thioharnstoff, insektizid wirksam sind (vergleiche US-PS 3 933 908).

Weiter ist bekannt, daß bestimmte Kupferverbindungen, wie z. B. Kupferoxychlorid, fungizide und bakterizide Eigenschaften aufweisen.

Es wurden nun die neuen N-Phenyl-N'-benzoyl-thioharnstoffe der Formel (I)

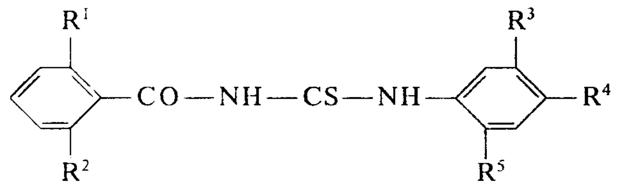

(I)

in welcher
$R^1$ für Halogen steht,
$R^2$ für Wasserstoff oder Halogen steht,
$R^3$ für Wasserstoff, Halogen oder Halogenalkyl steht,
$R^4$ für Halogen, Halogenalkyl, Halogenalkoxy, Halogenalkylthio oder Halogenalkylsulfonyl steht mit der Maßgabe, daß $R^3$ für Halogenalkyl steht, wenn $R^4$ für Halogen steht, und
$R^5$ für Wasserstoff oder Halogen steht,
gefunden.

Die neuen Verbindungen zeigen sehr gute Wirkungen als Schädlingsbekämpfungsmittel; insbesondere zeichnen sie sich durch eine hervorragende insektizide Wirksamkeit aus und sind außerdem fungizid und bakterizid wirksam.

Weiter wurde gefunden, daß man die neuen N-Phenyl-N'-benzoyl-thioharnstoffe der Formel (I) erhält, wenn man Benzoylisothiocyanate (Benzoylsenföle) der Formel II

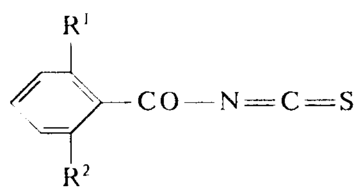

(II)

in welcher
$R^1$ und $R^2$ die oben angegebene Bedeutung haben,
mit substituierten Anilinen der Formel III

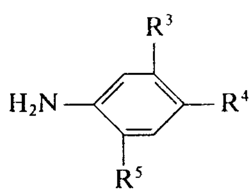

(III)

in welcher
$R^3$, $R^4$ und $R^5$ die oben angegebene Bedeutung haben,
gegebenenfalls unter Verwendung inerter Verdünnungsmittel, umsetzt.

Überraschenderweise zeigen die erfindungsgemäßen neuen N-Phenyl-N'-benzoyl-thioharnstoffe eine wesentlich bessere Wirksamkeit als Schädlingsbekämpfungsmittel, insbesondere eine wesentlich höhere insektizide Wirkung als die nächstliegenden bekannten Benzoyl-thioharnstoffe und eine wesentlich bessere bakterizide Wirkung als das bekannte Kupferoxychlorid. Die Produkte gemäß vorliegender Erfindung stellen somit eine wertvolle Bereicherung der Technik dar.

Gegenstand der Erfindung sind vorzugsweise N-Phenyl-N'-benzoyl-thioharnstoffe der Formel (I), worin
$R^1$ für Fluor, Chlor, Brom oder Jod steht,
$R^2$ für Wasserstoff, Fluor, Chlor, Brom oder Jod steht,
$R^3$ für Wasserstoff, Chlor oder Trifluormethyl steht,
$R^4$ für Chlor, Fluor- oder Chlorfluor-alkyl, Fluor- oder Chlorfluor-alkoxy, Fluor- oder Chlorfluor-alkylthio oder für Fluoralkylsulfonyl steht — wobei die Halogenalkylgruppen jeweils 1 oder 2

Kohlenstoffatome enthalten – mit der Maßgabe, daß $R^3$ für Trifluormethyl steht, wenn $R^4$ für Chlor steht, und

$R^5$ für Wasserstoff oder Chlor steht.

Verwendet man beispielsweise 2,6-Dichlorbenzoyl-isothiocyanat und 4-Trifluormethoxy-anilin als Ausgangsstoffe, so kann die Reaktion dieser Verbindungen durch folgendes Formelschema skizziert werden:

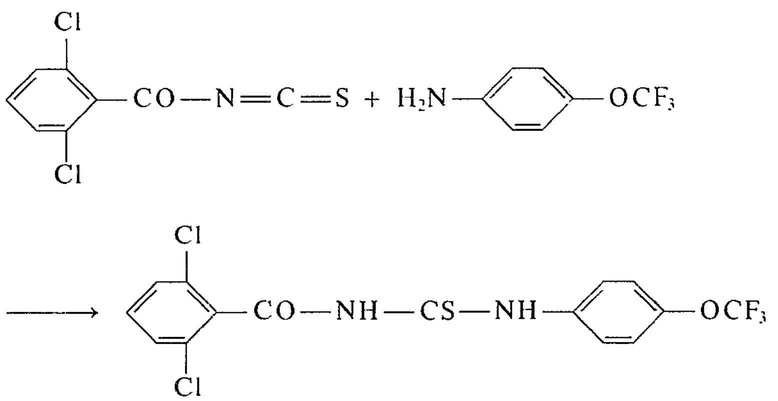

Die zu verwendenden Ausgangsstoffe sind durch die Formeln (II) und (III) definiert.

Vorzugsweise stehen darin $R^1$ bis $R^5$ für diejenigen Reste, welche bei der Definition der Reste $R^1$ bis $R^5$ in Formel (I) als bevorzugt genannt wurden.

Die als Ausgangsverbindungen zu verwendenden Benzoylisothiocyanate der Formel (II) sind bekannt oder nach literaturbekannten Verfahren herstellbar (vergleiche Houben–Weyl, Methoden der organischen Chemie, 4. Aufl., Band 9, S. 878–879, Georg Thieme Verlag, Stuttgart, 1955). Man erhält sie insbesondere in hoher Reinheit durch Umsetzung von Trimethylsilanylsenföl mit den entsprechenden substituierten Benzoylhalogeniden bei Temperaturen zwischen 100 und 250°C (vergleiche DE-AS 1 215 144).

Als Beispiele für die Benzoyl-isothiocyanate der Formel (II) seien genannt:

2-Fluor-, 2-Chlor-, 2-Brom- und 2-Jod-benzoyl-isothiocyanat,
2,6-Difluor- und 2,6-Dichlor-benzoyl-isothiocyanat sowie
2-Chlor-6-fluor-benzoyl-isothiocyanat.

Die als weitere Ausgangsstoffe zu verwendenden substituierten Aniline der Formel (III) sind ebenfalls bekannt oder nach bekannten Verfahren herstellbar (vergleiche z. B. J. Org. Chem., 29 [1964], 1–11 und US-PS 3 387 037).

Als Beispiele für die substituierten Aniline der Formel (III) seien genannt:

4-Trifluormethylanilin, 3,4-Bis-trifluormethyl-anilin,
2-Chlor-4-trifluormethyl-anilin, 3-Chlor-4-trifluormethylanilin,
4-Chlor-3-trifluormethyl-anilin, 4-Difluormethoxyanilin,
4-Trifluormethoxy- und 4-Trifluormethylthio-anilin,
2-Chlor- und 3-Chlor-4-trifluormethoxy-anilin,
2-Chlor- und 3-Chlor-4-trifluormethylthio-anilin,
4-Chlordifluormethoxy- und 4-Chlordifluormethylthio-anilin,
2-Chlor- und 3-Chlor-4-chlordifluormethoxy-anilin,
2-Chlor- und 3-Chlor-4-chlordifluormethylthio-anilin,
4-(2-Chlor-1,1,2-trifluor-äthoxy)- und 4-(2-Chlor-1,2,2-trifluoräthylthio)-anilin,
2-Chlor- und 3-Chlor-4-(2-chlor-1,1,2-trifluor-äthoxy)-anilin,
2-Chlor- und 3-Chlor-4-(2-chlor-1,1,2-trifluor-äthylthio)-anilin,
4-Trifluormethylsulfonyl-anilin sowie
2-Chlor- und 3-Chlor-4-trifluormethylsulfonyl-anilin.

Das Verfahren zur Herstellung der erfindungsgemäßen N-Phenyl-N'-benzoyl-thioharnstoffe wird bevorzugt unter Verwendung geeigneter Lösungs- oder Verdünnungsmittel durchgeführt. Als solche kommen praktisch alle inerten organischen Solventien in Frage. Hierzu gehören insbesondere aliphatische und aromatische, gegebenenfalls chlorierte Kohlenwasserstoffe, wie Benzin, Benzol, Toluol, Xylol, Methylenchlorid, Chloroform, Tetrachlorkohlenstoff, Chlorbenzol und o-Dichlorbenzol, Äther wie Diäthyl- und Dibutyläther, Tetrahydrofuran und Dioxan, Ketone wie Aceton, Methyläthyl-, Methylisopropyl- und Methylisobutylketon sowie Nitrile wie Acetonitril und Propionitril.

Die Reaktionstemperatur kann innerhalb eines größeren Bereichs variiert werden. Im allgemeinen arbeitet man zwischen 0 und 150°C, vorzugsweise bei 20 bis 100°C. Das erfindungsgemäße Verfahren wird im allgemeinen bei Normaldruck durchgeführt.

Zur Durchführung des erfindungsgemäßen Verfahrens werden die Ausgangsstoffe gewöhnlich in äquimolaren Mengen eingesetzt. Ein Überschuß der einen oder anderen Reaktionskomponente bringt

keine wesentlichen Vorteile. Die Umsetzung wird im allgemeinen in einem geeigneten Verdünnungsmittel durchgeführt und das Reaktionsgemisch wird eine oder mehrere Stunden bei der erforderlichen Temperatur gerührt. Danach läßt man das Reaktionsgemisch auf Raumtemperatur abkühlen und saugt vom auskristallisierten Produkt ab. Zur Charakterisierung dient der Schmelzpunkt.

Die erfindungsgemäßen N-Phenyl-N'-benzoyl-thioharnstoffe zeichnen sich durch eine hervorragende insektizide Wirksamkeit aus. Sie zeigen ferner eine sehr gute Wirksamkeit gegen phytopathogene Bakterien und Pilzkrankheiten.

Die Wirkstoffe eignen sich bei guter Pflanzenverträglichkeit und günstiger Warmblütertoxizität zur Bekämpfung von tierischen Schädlingen, insbesondere Insekten und Spinnentieren, die in der Landwirtschaft, in Forsten, im Vorrats- und Materialschutz sowie auf dem Hygienesektor vorkommen. Sie sind gegen normal sensible und resistente Arten sowie gegen alle oder einzelne Entwicklungsstadien wirksam. Zu den obenerwähnten Schädlingen gehören:

Aus der Ordnung der Isopoda z. B. Oniscus asellus, Armadillidium vulgare, Porcellio scaber.

Aus der Ordnung der Diplopoda z. B. Blaniulus guttulatus.

Aus der Ordnung der Chilopoda z. B. Geophilus carpophagus, Scutigera spec.

Aus der Ordnung der Symphyla z. B. Scutigerella immaculata.

Aus der Ordnung der Thysanura z. B. Lepisma saccharina.

Aus der Ordnung der Collembola z. B. Onychiurus armatus.

Aus der Ordnung der Orthoptera z. B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.

Aus der Ordnung der Dermaptera z. B. Forficula auricularia.

Aus der Ordnung der Isoptera z. B. Reticulitermes spp.

Aus der Ordnung der Anoplura z. B. Phylloxera vastatrix, Pemphigus spp., Pediculus humanus corporis, Haematopinus spp., Linognathus spp.

Aus der Ordnung der Mallophaga z. B. Trichodectes spp., Damalinea spp.

Aus der Ordnung der Thysanoptera z. B. Hercinothrips femoralis, Thrips tabaci.

Aus der Ordnung der Heteroptera z. B. Eurygaster spp., Dysdercus intermedius, Piesma quadrata, Cimex lectularius, Rhodnius prolixus, Triatoma spp.

Aus der Ordnung der Homoptera z. B. Aleurodes brassicae, Bemisia tabaci, Trialeurodes vaporariorum, Aphis gossypii, Brevicoryne brassicae, Cryptomyzus ribis, Doralis fabae, Doralis pomi, Eriosoma lanigerum, Hyalopterus arundinis, Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp. Psylla spp.

Aus der Ordnung der Lepidoptera z. B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lamantria spp. Bucculatrix thurberiella, Phyllocnistis citrella, Agrotis spp., Euxoa spp., Feltia spp. Earias insulana, Heliothis spp., Laphygma exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Cacoecia podana, Capua reticulana, Choristoneura fumiferana, Clysia ambiguella, Homona magnanima, Tortrix viridana.

Aus der Ordnung der Coleoptera z. B. Anobium punctatum, Rhizopertha dominica, Bruchidius obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica.

Aus der Ordnung der Hymenoptera z. B. Diprion spp., Hoplocampa spp., Lasius spp., Monomorium pharaonis, Vespa spp.

Aus der Ordnung der Diptera z. B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.

Aus der Ordnung der Siphonaptera z. B. Xenopsylla cheopis, Ceratophyllus spp.

Aus der Ordnung der Arachnida z. B. Scorpio maurus, Latrodectus mactans.

Aus der Ordnung der Acarina z. B. Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp.

Die Wirkstoffe können in die üblichen Formulierungen übergeführt werden, wie Lösungen, Emulsionen, Suspensionen, Pulver, Schäume, Pasten, Granulate, Aerosole, wirkstoffimprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u. ä., sowie ULV-Kalt- und Warmnebel-Formulierungen.

Diese Formulierungen werden in bekannter Weise hergestellt, z. B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z. B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chloräthylene oder Methylenchlorid, aliphatische Kohlenwasserstoffe, wie Cyclohexan oder Paraffine, z. B. Erdölfraktionen, Alkohole, wie Butanol oder Glycol sowie deren Äther und Ester, Ketone, wie Aceton, Methyläthylketon, Methylisobutylketon oder Cyclohexanon, stark polare Lösungsmittel, wie Dimethylformamid und Dimethylsulfoxid sowie Wasser; mit verflüssigten gasförmigen Streckmitteln oder Trägerstoffen sind solche Flüssigkeiten gemeint, welche bei normaler Temperatur und unter Normaldruck gasförmig sind, z. B. Aerosol-Treibgas, wie Halogenkohlenwasserstoffe sowie Butan, Propan, Stickstoff und Kohlendioxid; als feste Trägerstoffe kommen in Frage:

z. B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate;

als feste Trägerstoffe für Granulate kommen in Frage:

z. B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel;

als Emulgier- und/oder schaumerzeugende Mittel kommen in Frage:

z. B. nichtionogene und anionische Emulgatoren, wie Polyoxyäthylen-Fettsäure-Ester, Polyoxyäthylen-Fettalkohol-Äther, z. B. Alkylarylpolyglykol-äther, Alkylsulfonate, Alkylsulfate, Arylsulfonate sowie Eiweißhydrolysate;

als Dispergiermittel kommen in Frage:

z. B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat.

Es können Farbstoffe wie anorganische Pigmente, z. B. Eisenoxid, Titanoxid, Ferrocyanblau und organische Farbstoffe, wie Alizarin-, Azol-Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90%.

Die Anwendung der erfindungsgemäßen Wirkstoffe erfolgt in Form ihrer handelsüblichen Formulierungen und/oder den aus diesen Formulierungen bereiteten Anwendungsformen.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 100 Gew.-% Wirkstoff, vorzugsweise zwischen 0,01 und 10 Gew.-% liegen.

Die Anwendung geschieht in einer den Anwendungsformen angepaßten üblichen Weise.

Bei der Anwendung gegen Hygiene- und Vorratsschädlinge zeichnen sich die Wirkstoffe durch eine hervorragende Residualwirkung auf Holz und Ton sowie durch eine gute Alkalistabilität auf gekälkten Unterlagen aus.

Die Anwendung der erfindungsgemäßen Wirkstoffe geschieht im Veterinärsektor in bekannter Weise, wie durch orale Anwendung in Form von beispielsweise Tabletten, Kapseln, Tränken, Granulaten, durch dermale Anwendung in Form beispielsweise des Tauchens (Dippen), Sprühens, (Sprayen), Aufgießens (pour-on an spot-on) und das Einpudern sowie durch parenterale Anwendung in Form beispielsweise der Injektion.

Erfindungsgemäße Verbindungen wurden in den folgenden Beispielen A–C mit der eingangs erwähnten Verbindung N-(2,6-Dichlorbenzoyl)-N'-(3,4-dichlorphenyl)-thioharnstoff (vgl. US-PS 3 933 908) und im folgenden Beispiel E mit Kupferoxidchlorid geprüft.

Beispiel A

Plutella-Test

Lösungsmittel: 3 Gewichtsteile Dimethylformamid
Emulgator: 1 Gewichtsteil Alkylarylpolyglykoläther

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Raupen der Kohlschabe (Plutella maculipennis) besetzt, solange die Blätter noch feucht sind.

Nach der gewünschten Zeit wird die Abtötung bestimmt.

Bei diesem Test zeigen z. B. die folgenden Verbindungen der Herstellungsbeispiele überlegene Wirksamkeit gegenüber dem Stand der Technik:

11, 51, 16, 6, 39, 50, 2, 13, 23, 8,
30, 40, 22, 5, 46, 3, 9, 7, 28,
18, 10, 21

Beispiel B

Laphygma-Test

Lösungsmittel: 3 Gewichtsteile Dimethylformamid
Emulgator: 1 Gewichtsteil Alkylarylpolyglykoläther

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Raupen des Eulenfalters (Laphygma frugiperda) besetzt, solange die Blätter noch feucht sind.

Nach der gewünschten Zeit wird die Abtötung bestimmt.

Bei diesem Test zeigen z. B. die folgenden Verbindungen der Herstellungsbeispiele überlegene Wirksamkeit gegenüber dem Stand der Technik:

4, 38, 52, 1, 53, 43, 54, 41

Beispiel C

Mückenlarven-Test

Testtiere: Aedes aegypti 2. Larvenstadium
Lösungsmittel: 99 Gewichtsteile Aceton
Emulgator: 1 Gewichtsteil Benzylhydroxydiphenylether

Zur Herstellung einer zweckmäßigen Wirkstoffzubereitung löst man 2 Gewichtsteile Wirkstoff in 1000 Volumteilen Lösungsmittel, das Emulgator in der oben angegebenen Menge enthält. Die so erhaltene Lösung wird mit Wasser auf die gewünschten geringeren Konzentrationen verdünnt.

Man füllt die wäßrigen Wirkstoffzubereitungen der gewünschten Konzentration in Gläser und setzt anschließend etwa 25 Mückenlarven in jedes Glas ein.

Nach 24 Stunden wird der Abtötungsgrad bestimmt.

Bei diesem Test zeigen z. B. die folgenden Verbindungen der Herstellungsbeispiele überlegene Wirkung gegenüber dem Stand der Technik:

30, 4, 1, 6, 7, 5, 36, 37, 53, 13, 8,
24, 9, 10, 29, 11, 44, 45, 19, 20, 22,
23, 3, 16

## Beispiel D

Test mit parasitierenden Fliegenlarven (Lucilia cuprina res.)

Emulgator: 80 Gewichtsteile Alkylarylpolyglykoläther

Zur Herstellung einer zweckmäßigen Wirkstoffzubereitung vermischt man 20 Gew.-Teile der betreffenden aktiven Substanz mit der angegebenen Menge des Emulgators und verdünnt das so erhaltene Gemisch mit Wasser auf die gewünschte Konzentration. Etwa 20 Fliegenlarven (Lucilia cuprina) werden in ein mit Wattestopfen entsprechender Größe beschicktes Teströhrchen gebracht, welche ca. 3 ml einer 20%igen Eigelbpulver-Suspension in Wasser enthält. Auf diese Eigelbpulver-Suspension werden 0,5 ml der Wirkstoffzubereitung gebracht. Nach 24 Stunden wird der Abtötungsgrad bestimmt.

Bei diesem Test zeigen z. B. die folgenden Verbindungen der Herstellungsbeispiele 2, 8, 11, 19, 37, 39, 40, 41, 42, 46, 52 und 53 eine gute Wirkung.

## Beispiel E

Bakterien-Test/Xanthomonas oryzae

| | |
|---|---|
| Lösungsmittel: | 11,75 Gewichtsteile Aceton |
| Dispergiermittel: | 0,75 Gewichtsteile Alkylarylpolyglykoläther |
| Wasser: | 987,50 Gewichtsteile |
| Andere Zusätze: | — Gewichtsteile |

Man vermischt die für die gewünschte Wirkstoffkonzentration in der Spritzflüssigkeit nötige Wirkstoffmenge mit der angegebenen Menge des Lösungsmittels und des Dispergiermittels und verdünnt das Konzentrat mit der angegebenen Menge Wasser.

Mit der Spritzflüssigkeit bespritzt man 30 etwa 40 Tage alte Reispflanzen bis zur Tropfnässe. Die Pflanzen verbleiben bis zum Abtrocknen in einem Gewächshaus bei Temperaturen von 22 bis 24°C und einer relativen Luftfeuchtigkeit von etwa 70%. Danach werden Nadeln in eine wäßrige Bakteriensuspension von Xanthomonas eryzae getaucht und die Pflanzen durch Anstechen der Blätter inokuliert. Die Pflanzen stehen nach der Inokulation 24 Stunden bei 100% relativer Luftfeuchtigkeit und danach in einem Raum bei 26 bis 28°C und 80% relativer Luftfeuchtigkeit. 10 Tage nach der Inokulation wird der Befall bei allen durch Stich verletzten, inokulierten Blätter von vorher mit Präparat behandelten Pflanzen.

Bei diesem Test zeigen z. B. die folgenden Verbindungen der Herstellungsbeispiele überlegene Wirkung gegenüber dem Stand der Technik:

27, 4, 38, 14, 29

# Herstellungsbeispiele

## Beispiel 1

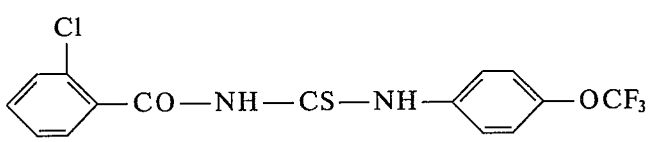

Zu einer Lösung von 5,4 g (0,03 Mol) 4-Trifluormethoxyanilin in 50 ml Toluol werden bei 60°C 6 g 2-Chlorbenzoylsenföl ($Kp._2$ 105–106°C, n =1,6495) in 20 ml Toluol zugetropft. Der Ansatz wird eine Stunde bei 80°C gerührt. Nach dem Abkühlen auf Raumtemperatur wird das kristalline Produkt abgesaugt. Es besitzt einen Schmelzpunkt von 197°C. Seine Identifizierung erfolgte durch Elementaranalyse und NMR-spektroskopisch. Die Ausbeute beträgt 6,1 g (54% der Theorie) an N-(2-Chlorbenzoyl)-N'-(4-trifluormethoxy-phenyl)-thioharnstoff.

## Beispiel 2

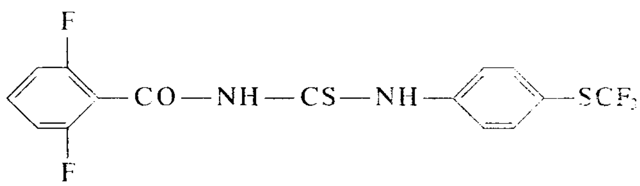

Eine Lösung von 5,8 g 4-Trifluormethylmercaptoanilin (0,03 Mol) in 60 ml Toluol wird mit einer Lösung von 6 g 2,6-Difluorbenzoylsenföl ($Kp._1$ 98°C, n =1,5872) in 20 ml Toluol versetzt und eine Stunde bei 80°C gerührt. Beim Abkühlen fällt das Produkt aus. Es wird abgesaugt und mit Petroläther gewaschen. Die Substanz schmilzt bei 207°C. Die Identifizierung erfolgte durch Elementaranalyse und NMR-spektroskopisch. Ausbeute 9,6 g (81,5% der Theorie) an N-(2,6-Difluorbenzoyl)-N'-(4-trifluormethylthio-phenyl)-thioharnstoff.

Analog wurden folgende Verbindungen der allgemeinen Formel

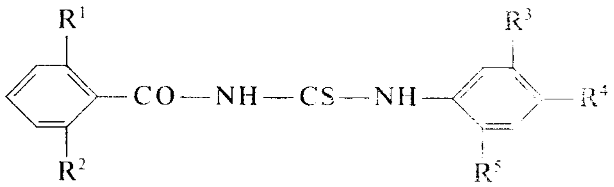

(I)

erhalten:

| Beispiel Nr. | $R^1$ | $R^2$ | $R^3$ | $R^4$ | $R^5$ | Schmelzpunkt (°C) | Ausbeute (% der Theorie) |
|---|---|---|---|---|---|---|---|
| 3 | Br | H | H | $OCF_2Cl$ | H | 163 | 84,5 |
| 4 | Cl | H | H | $CF_3$ | H | 197 | 74 |
| 5 | Cl | H | H | $OCF_2Cl$ | H | 157 | 36 |
| 6 | Cl | H | Cl | $SCF_3$ | H | 128 | 45,5 |
| 7 | Cl | H | Cl | $OCF_2-CHFCl$ | H | 160 | 79,5 |
| 8 | Cl | H | Cl | $OCF_3$ | H | 158 | 72,5 |
| 9 | Cl | H | H | $OCF_2-CHFCl$ | H | 160 | 72 |
| 10 | Cl | H | Cl | $SCF_2Cl$ | H | 118 | 55 |
| 11 | Cl | H | Cl | $CF_3$ | H | 173 | 74,5 |
| 12 | Br | H | H | $OCF_3$ | H | 176 | 71,5 |
| 13 | Br | H | H | $SCF_3$ | H | 158 | 50 |
| 14 | Br | H | H | $CF_3$ | H | 200 | 80 |
| 15 | Br | H | $CF_3$ | $CF_3$ | H | 163 | 95,5 |
| 16 | Br | H | Cl | $CF_3$ | H | 187 | 64,5 |
| 17 | Br | H | H | $CF_3$ | Cl | 149 | 89,5 |
| 18 | Br | H | Cl | $OCF_2-CHFCl$ | H | 164 | 90 |
| 19 | Br | H | Cl | $SCF_2Cl$ | H | 126 | 84 |

Fortsetzung

| Beispiel Nr. | $R^1$ | $R^2$ | $R^3$ | $R^4$ | $R^5$ | Schmelzpunkt (°C) | Ausbeute (% der Theorie) |
|---|---|---|---|---|---|---|---|
| 20 | Br | H | H | $OCF_2-CHFCl$ | H | 170 | 95,5 |
| 21 | Br | H | H | $SO_2CF_3$ | H | 165 | 91 |
| 22 | Br | H | Cl | $OCF_3$ | H | 168 | 97 |
| 23 | Br | H | Cl | $SCF_3$ | H | 138 | 88,5 |
| 24 | Cl | H | $CF_3$ | $CF_3$ | H | 148 | 80,5 |
| 25 | Br | H | H | $OCF_2CHFCl$ | Cl | 158 | 84,5 |
| 26 | J | H | H | $SCF_3$ | H | 174,5 | 76 |
| 27 | F | H | H | $CF_3$ | H | 116–120 | 85,5 |
| 28 | F | H | Cl | $OCF_2-CHFCl$ | H | 98–100 | 62 |
| 29 | Cl | H | $CF_3$ | Cl | H | 180 | 81 |
| 30 | Cl | Cl | H | $OCF_3$ | H | 215,5 | 66 |
| 31 | Cl | Cl | Cl | $OCF_2-CHFCl$ | H | 207,5 | 71,5 |
| 32 | Cl | Cl | H | $SO_2CF_3$ | H | 232 | 77,5 |
| 33 | Cl | Cl | H | $OCF_2-CHFCl$ | H | 210 | 69 |
| 34 | Cl | Cl | Cl | $SCF_2CL$ | H | 188 | 62,5 |
| 35 | Cl | Cl | Cl | $OCF_3$ | H | 221 | 76 |
| 36 | Cl | Cl | H | $OCF_2Cl$ | H | 201 | 77,5 |
| 37 | Cl | F | H | $OCF_3$ | H | 187 | 76,5 |
| 38 | Cl | F | H | $CF_3$ | H | 226 | 78 |
| 39 | Cl | F | H | $SCF_3$ | H | 180 | 57 |
| 40 | Cl | F | Cl | $OCF_3$ | H | 198 | 91 |
| 41 | Cl | F | H | $OCF_2-CHFCl$ | H | 195 | 90 |
| 42 | Cl | F | Cl | $SCF_2Cl$ | H | 163 | 76,5 |
| 43 | Cl | F | H | $SO_2CF_3$ | H | 208 | 88 |
| 44 | Cl | F | H | $CF_3$ | Cl | 202 | 90 |
| 45 | Cl | F | Cl | $OCF_2-CHFCl$ | H | 158 | 95,5 |
| 46 | Cl | F | H | $OCF_2Cl$ | H | 175 | 94,5 |
| 47 | Cl | F | $CF_3$ | $CF_3$ | H | 215 | 89 |
| 48 | Cl | F | $CF_3$ | Cl | H | 230 | 94,5 |

Fortsetzung

| Beispiel Nr. | $R^1$ | $R^2$ | $R^3$ | $R^4$ | $R^5$ | Schmelzpunkt (°C) | Ausbeute (% der Theorie) |
|---|---|---|---|---|---|---|---|
| 49 | Cl | F | H | $OCF_2CHFCl$ | Cl | 182 | 85 |
| 50 | Cl | F | Cl | $SCF_3$ | H | 177 | 94,5 |
| 51 | Cl | F | Cl | $CF_3$ | H | 215 | 86,5 |
| 52 | F | F | H | $CF_3$ | H | 210 | 90,5 |
| 53 | F | F | H | $OCF_3$ | H | 180 | 97,5 |
| 54 | Cl | F | H | $OCHF_2$ | H | 179 | 95,0 |
| 55 | F | F | Cl | $SCF_2Cl$ | H | 151 | 82,0 |
| 56 | F | F | H | $OCF_2CHFCl$ | H | 182 | 89,0 |
| 57 | F | F | $CF_3$ | $CF_3$ | H | 186 | 92,5 |
| 58 | F | F | H | $OCHF_2$ | H | 174 | 93,5 |

Die nachstehend aufgeführten als Ausgangsverbindungen verwendeten Benzoyl-isothiocyanate der Formel II

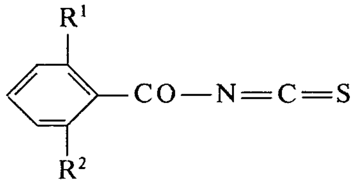 (II)

wurden analog Beispiel 1 der DE-AS 1 215 144 hergestellt:

| Beispiel | $R^1$ | $R^2$ | Siedepunkt (°C, bei 2 mbar) | Brechungsindex: | Ausbeute (% der Theorie) |
|---|---|---|---|---|---|
| a | F | H | 104 | $n_D^{20}$: 1,6200 | 79 |
| b | Br | H | 140 | $n_D^{20}$: 1,6702 | 85,5 |
| c | J | H | 148 | | 76,5 |
| d | Cl | H | 106 | $n_D^{20}$: 1,6495 | 87,5 |
| e | Cl | F | 104 | $n_D^{20}$: 1,6018 | 93 |
| f | Cl | Cl | 114 | $n_D^{20}$: 1,6205 | 77 |
| g | F | F | 98 | $n_D^{20}$: 1,5872 | 88 |

**Patentansprüche**

1. N-Phenyl-N'-benzoyl-thioharnstoffe der Formel I

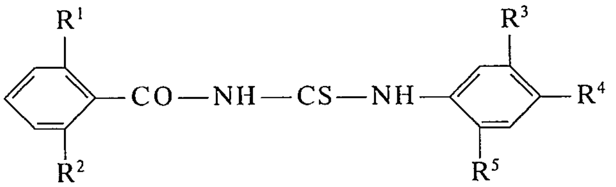

(I)

in welcher
$R^1$ für Halogen steht,
$R^2$ für Wasserstoff oder Halogen steht,
$R^3$ für Wasserstoff, Halogen oder Halogenalkyl steht,
$R^4$ für Halogen, Halogenalkyl, Halogenalkoxy, Halogenalkylthio oder Halogenalkylsulfonyl steht mit der Maßgabe, daß $R^3$ für Halogenalkyl steht, wenn $R^4$ für Halogen steht, und
$R^5$ für Wasserstoff oder Halogen steht.

2. Verfahren zur Herstellung von N-Phenyl-N'-benzoyl-thioharnstoffen der Formel (I), dadurch gekennzeichnet, daß man Benzoylisothiocyanate der Formel II

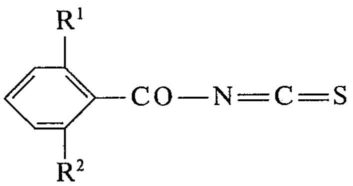

(II)

in welcher
$R^1$ und $R^2$ die oben angegebene Bedeutung haben,
mit substituierten Anilinen der Formel III

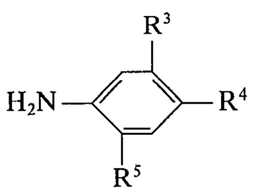

(III)

in welcher
$R^3$ und $R^4$ und $R^5$ die oben angegebene Bedeutung haben,
gegebenenfalls unter Verwendung inerter Verdünnungsmittel, umsetzt.

3. Schädlingsbekämpfungsmittel, gekennzeichnet durch einen Gehalt an mindestens einem N-Phenyl-N'-benzoyl-thioharnstoff der Formel (I).

4. Verwendung von N-Phenyl-N'-benzoyl-thioharnstoffen der Formel (I) zur Bekämpfung von Insekten, Spinnentieren und/oder pflanzenpathogenen Pilzen und Bakterien.

5. Verfahren zur Bekämpfung von Insekten, Spinnentieren und/oder pflanzenpathogenen Pilzen und Bakterien, dadurch gekennzeichnet, daß man N-Phenyl-N'-benzoyl-thioharnstoffe der Formel (I) auf Insekten, Spinnentiere und/oder pflanzenpathogenen Pilzen und Bakterien und/oder ihren Lebensraum einwirken läßt.

6. Verfahren zur Herstellung von Schädlingsbekämpfungsmitteln, dadurch gekennzeichnet, daß man N-Phenyl-N'-benzoyl-thioharnstoffe der Formel (I) mit Streckmitteln und/oder oberflächenaktiven Mitteln vermischt.

**Claims**

1. N-Phenyl-N′-benzoyl-thioureas of the formula I

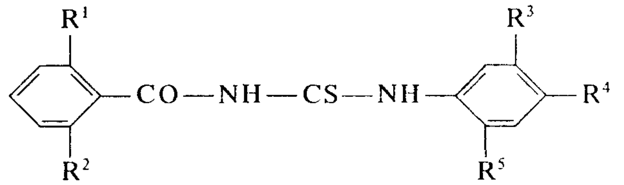

(I)

in which
$R^1$ represents halogen,
$R^2$ represents hydrogen or halogen,
$R^3$ represents hydrogen, halogen or halogenoalkyl,
$R^4$ represents halogen, halogenoalkyl, halogenoalkoxy, halogenoalkylthio or halogenoalkylsulphonyl, with the proviso that $R^3$ represents halogenoalkyl if $R^4$ represents halogen, and
$R^5$ represents hydrogen or halogen.

2. Process for the preparation of N-phenyl-N′-benzoyl-thioureas of the formula (I), characterised in that benzoyl isothiocyanates of the formula II

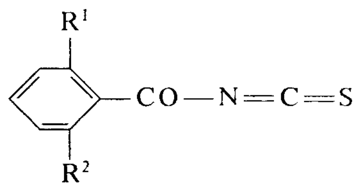

(II)

in which
$R^1$ and $R^2$ have the meaning indicated above,
are reacted with substituted anilines of the formula III

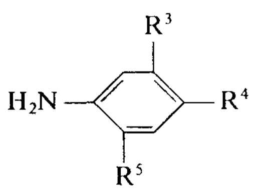

(III)

in which
$R^3$, $R^4$ and $R^5$ have the meaning indicated above,
if appropriate using inert diluents.

3. Agents for combating pests, characterised in that they contain at least one N-phenyl-N′-benzoyl-thiourea of the formula (I).

4. Use of N-phenyl-N′-benzoyl-thioureas of the formula (I) for combating insects, arachnidae and/or phytopathogenic fungi and bacteria.

5. Process for combating insects, arachnidae and/or phytopathogenic fungi and bacteria, characterised in that N-phenyl-N′-benzoyl-thioureas of the formula (I) are allowed to act on insects, arachnidae and/or phytopathogenic fungi and bacteria and/or their environment.

6. Process for the preparation of agents for combating pests, characterised in that N-phenyl-N′-benzoyl-thioureas of the formula (I) are mixed with extenders and/or surfaceactive agents.

**Revendications**

1. N-phényl-N′-benzoyl-thiourées de formule I

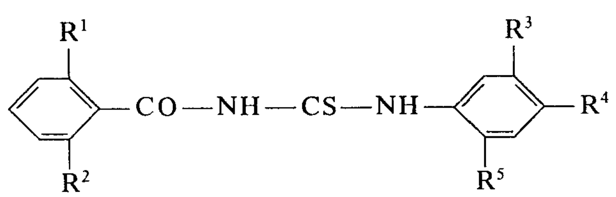

(I)

dans laquelle:

$R^1$ est un atome d'halogène;

$R^2$ est un atome d'hydrogène ou d'halogène;

$R^3$ est un atome d'hydrogène ou d'halogène ou un groupe halogénoalkyle;

$R^4$ est un atome d'halogène ou un groupe halogéno-alkyle, halogéno-alcoxy, halogénoalkylthio ou halogénoalkylsulfonyle, étant bien entendu que $R^3$ est un groupe halogénoalkyle lorsque $R^4$ est un atome d'halogène; et

$R^5$ est un atome d'hydrogène ou d'halogène.

2. Procédé pour produire des N-phényl-N'-benzoyl-thiourées de formule (I), caractérisé en ce qu'on fait réagir des isothiocyanates de benzoyles de formule II:

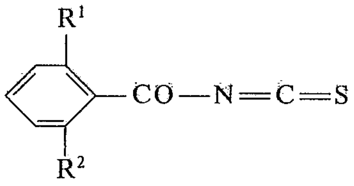

(II)

(dans laquelle $R^1$ et $R^2$ ont le sens indiqué ci-dessus) avec des anilines substituées de formule III:

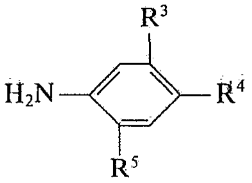

(III)

(dans laquelle $R^3$ et $R^4$ et $R^5$ ont le sens indiqué ci-dessus) en utilisant éventuellement des solvants inertes.

3. Pesticide, caractérisé en ce qu'il contient au moins une N-phényl-N'-benzoyl-thiourée de formule (I).

4. Application des N-phényl-N'-benzoyl-thiourées de formule (I) pour lutter contre des insectes, des araignées et éventuellement ou en variante des champignons et bactéries pathogènes pour les plantes.

5. Procédé pour combattre les insectes, les araignées et/ou les champignons ou bactéries pathogènes pour les plantes, caractérisé en ce qu'on fait agir des N-phényl-N'-benzoyl-thiourées de formule (I) sur les insectes, les araignées et/ou les champignons et bactéries pathogènes pour les plantes et/ou sur leur espace vital.

6. Procédé pour produire des pesticides, caractérisé en ce qu'on mélange des N-phényl-N'-benzoyl-thiourées de formule (I) avec des agents d'allongement et/ou des agents tensioactifs.